# EUROPÄISCHE PATENTSCHRIFT

(11) **EP 0 931 785 B1**
(45) Veröffentlichungstag und Bekanntmachung des Hinweises auf die Patenterteilung: **17.04.2002**
(21) Anmeldenummer: 98124382.7
(22) Anmeldetag: 21.12.1998
(51) Int. Cl.: C07C 29/70

(54) **Verfahren zur Herstellung niederer und höherer Alkalialkoholate, insbesondere Kaliumtertiärbutylat, durch mikroheterogene Katalyse**
Method for the preparation of lower and higher alkali metal alcoholates, particularly potassium tertiary butylate, by microheterogenous catalysis
Procédé pour la préparation catalytique d'alcoolats inférieurs et supérieurs de métaux alcalins, particulièrement tertiaire butylate de potasse, par catalyse microhétérogène

(30) Priorität: 21.01.1998 DE 19802013
(43) Veröffentlichungstag der Anmeldung: 28.07.1999
(73) Patentinhaber: Degussa AG, 40474 Düsseldorf (DE)
(72) Erfinder: Hamann, Carl Heinz Prof., 26939 Ovelgönne (DE); Helling, Jörg, 26121 Oldenburg (DE); Schmittinger, Peter Dr., 82008 Unterhaching (DE)

(56) Entgegenhaltungen:
- EP-A- 0 810 193
- US-A- 5 262 133

## Beschreibung

Die Erfindung betrifft ein Verfahren zur mikroheterogen-katalytischen Herstellung von Alkalialkoholaten, insbesondere von Kaliumtertiärbutylat aus Alkaliamalgamen und Alkoholen.

Alkalialkoholate, insbesondere solche, deren Alkoholkomponente bis zu 4 Kohlenstoffatome enthält, sind wertvolle Chemikalien. Sie werden z.B. als Katalysatoren bei der Synthese vieler organischer Verbindungen verwendet. Dabei haben vorzugsweise die Alkoholate des Natriums und des Kaliums praktische Bedeutung erlangt. Zur Darstellung von Alkalialkoholaten sind mehrere Methoden bekannt (F.A. Dickes, Ber. Dtsch. Chem. Ges. 63, 2753 [1930]. So enthalten Lösungen von Alkalihydroxiden in einem Alkohol Alkalialkoholat im Gleichgewicht. Durch z.B. destillative Entfernung des in diesem Gleichgewicht befindlichen Wassers gelangt man zu reinen Alkalialkoholaten. Besonders bei niedrig siedenden Alkoholen wird für diese Art der Gleichgewichtsverschiebung jedoch sehr viel Energie benötigt.

Auf direktem Wege kommt man zu reinen Alkalialkoholaten, indem man ein Alkalimetall in dem entsprechenden Alkohol auflöst. Dabei reagieren Natrium und Kalium mit niederen aliphatischen Alkoholen, wie Methanol und Ethanol, stürmisch unter Wasserstoffentwicklung. Höhere Alkoholate, wie z.B. Propanole und Butanole, setzt man vorzugsweise oberhalb des Schmelzpunktes der Alkalimetalle, gegebenenfalls unter Druck und Rühren, mit diesen um.

Alkalimetall als Ausgangsstoff ist jedoch teuer. Wirtschaftlicher ist es, als Alkaliquelle das flüssige, bei der Chloralkali-Elektrolyse nach dem Quecksilber-Verfahren anfallende Alkaliamalgam einzusetzen.

Die Umsetzung von Alkaliamalgam mit Alkoholen zu Alkoholaten sowie die Verwendung von Katalysatoren für diese Reaktion sind bekannt.

Mit dem Verfahren nach der EP-A 0 177 768 gelang es, die Reaktion zwischen dem Amalgam und dem Alkohol schon recht schnell zu führen. Es wird für die Reaktion eine Schüttung aus stückigem Anthrazit verwendet, deren Oberfläche mit Schwermetalloxid oder einer Mischung aus Schwermetalloxiden belegt ist. Amalgam und Alkohol werden nach dem Gegenstromprinzip kontinuierlich eingespeist, die Produkte kontinuierlich abgezogen.

Der damit beschriebene Stand der Technik war allerdings nach wie vor unbefriedigend. Z.B. werden nur 60 bis 80 % des mit dem Amalgam eingetragenen Natriums mit Methanol umgesetzt. Eine Zehrung von bis zu 100 % im Falle der Herstellung von MeONa wird erst erreicht, wenn man der DE 196 21 466 folgt.

US 5,262,133 betrifft die Herstellung von Alkalialkoholaten durch Umsetzung von Natrium amalgam und C₁-C₄-Alkoholen in Gegenwart eines auf einem Träger aufgebrachten Wolframcarbid-Katalysators.

Auch jetzt aber ist die Herstellung von z.B. Kaliumtertiärbutylat nur mit aus wirtschaftlicher Sicht noch unbefriedigender Raum-Zeit-Ausbeute möglich, selbst wenn man die Umsetzung im Autoklaven bei 140 °C und 7 bar führt.

Es bestand somit die Aufgabe, ein Verfahren zu entwickeln, welches die angeführten Nachteile behebt. Diese Aufgabe wurde gemäß der Patentansprüche gelöst.

Es wurde ein Verfahren zur katalytischen Herstellung von Alkalialkoholaten aus Alkaliamalgamen und Alkoholen gefunden, in dem die Umsetzung in Gegenwart von Pulverkatalysatoren aus Übergangsmetall-Carbiden, -Nitriden und -Carbonitriden durchgeführt wird. Besonders eignen sich die Metalle Molybdän und Wolfram und von diesen Metallen besonders die Carbide. Beim Einsatz von z.B. Mo₂C oder WC gelang überraschenderweise die Umsetzung selbst für tertiäre Alkohole befriedigend schnell (Mikroheterogene Katalyse).

Die Pulverkatalysatoren werden mit einem mittleren Korndurchmesser von 1 bis 10 µm, vorzugsweise 2 µm eingesetzt.

Für das erfindungsgemäße Verfahren eignen sich besonders lineare oder verzweigte aliphatische Alkohole mit 1 bis 5 Kohlenstoffatomen.

### Beispiel 1

In einem 2 000 ml Dreihalskolben mit Heizhaube, KPG-Rührer, Rückflußkühler und N₂-Einleitung werden 200 g tert-Butanol mit 2 kg Kaliumamalgam (0,32 Gew.-% K) und 20 g Mo₂C-Pulver versetzt und unter Rückfluß erhitzt. Nach ca. 20 Stunden Reaktionszeit und anschließender Filtration der alkalischen Phase wurden ca. 200 ml Kaliumtertiärbutylat-Lösung mit einem KTB-Gehalt von 14 % und einem KOH-Gehalt von 1,4 % erhalten.

### Beispiel 2

In einem 2 000 ml Rundkolben wurden 2,7 kg Kaliumamalgam mit 340 g tert-Butanol und 20 g Wolframcarbidpulver für 17 h unter Rückfluß erhitzt. Das Amalgam setzte sich zu etwa 38 % um. Es entsteht eine alkoholische Phase mit einem KTB-Gehalt von 7,2 Gew.-% und einem KOH-Gehalt von 1,6 Gew.-%.

### Beispiel 3

In einem Rührreaktor mit Wassermantel, Rückflußkühler, N₂-Einleitung und Magnetrührung werden bei 60 °C 100 g Natriumamalgam (0,2 Gew.-% Na) mit 100 g n-Amylalkohol in Gegenwart von 3 g Mo₂C-Pulver (2 µm) umgesetzt. Nach 3 h hat sich die gesamte Alkalimenge umgesetzt.

### Beispiel 4

In einer dem Beispiel 3 entsprechenden Versuchsanordnung setzten sich 100 g Methanol mit 100 g Natriumamalgam (0,2 Gew.-% Na) in Gegenwart von 1,2 g Mo₂C-Pulver (2 µm) binnen 1,7 Minuten vollständig um.

## Patentansprüche

1. Verfahren zur katalytischen Herstellung von Alkalialkoholaten aus Alkaliamalgamen und Alkoholen,
**dadurch gekennzeichnet,**
**daß** die Umsetzung in Gegenwart von Katalysatoren aus Übergangsmetall-Carbiden, -Nitriden oder -Carbonitriden in Pulverform geführt wird.

2. Verfahren nach Anspruch 1,
**dadurch gekennzeichnet,**
**daß** als Übergangsmetall Molybdän verwendet wird.

3. Verfahren nach Anspruch 1,
**dadurch gekennzeichnet,**
**daß** als Übergangsmetall Wolfram verwendet wird.

4. Verfahren nach Anspruch 1,
**dadurch gekennzeichnet,**
**daß** als Pulverkatalysator Molybdäncarbid verwendet wird.

5. Verfahren nach Anspruch 1,
**dadurch gekennzeichnet,**
**daß** als Pulverkatalysator Wolframcarbid verwendet wird.

6. Verfahren nach Anspruch 1,
**dadurch gekennzeichnet,**
**daß** die Pulverkatalysatoren mit einem mittleren Korndurchmesser von 1 bis 10 µm verwendet werden.

7. Verfahren nach Anspruch 1,
**dadurch gekennzeichnet,**
**daß** als Alkoholkomponente aliphatische Alkohole mit 1 bis 5 Kohlenstoffatomen umgesetzt werden.

## Revendications

1. Procédé de fabrication catalytique d'alcoolates alcalins
à partir d'amalgames alcalins et d'alcools,
**caractérisé par le fait que**
la conversion est effectuée en présence de catalyseurs
de métal de transition carbures, nitrures ou carbonitrures
sous forme pulvérulente.

2. Procédé selon la revendication 1,
**caractérisé par le fait**
**que** le métal de transition utilisé est du molybdène.

3. Procédé selon la revendication 1,
**caractérisé par le fait**
**que** le métal de transition utilisé est du tungstène.

4. Procédé selon la revendication 1,
**caractérisé par le fait**
**que** le catalyseur pulvérulent est du carbure de molybdène.

5. Procédé selon la revendication 1,
**caractérisé par le fait**
**que** le catalyseur pulvérulent utilisé est du carbure de tungstène.

6. Procédé selon la revendication 1,
**caractérisé par le fait**
**que** les catalyseurs pulvérulents utilisés ont un diamètre de grain moyen de 1 à 10 µm.

7. Procédé selon la revendication 1,
**caractérisé par le fait**
**que** des alcools aliphatiques comprenant 1 à 5 atomes de carbones sont transformés en tant que composante alcool.

## Claims

1. Process for the catalytic production of alkali alcoholates from alkali amalgams and alcohols, **characterized in that** the conversion is carried out in the presence of catalysts produced from transition metal carbides, nitrides or carbonitrides in powder form.

2. Process as claimed in Claim 1, **characterized in that** molybdenum is used as the transition metal.

3. Process as claimed in Claim 1, **characterized in that** tungsten is used as the transition metal.

4. Process as claimed in Claim 1, **characterized in that** molybdenum carbide is used as the powder catalyst.

5. Process as claimed in Claim 1, **characterized in that** tungsten carbide is used as the powder catalyst.

6. Process as claimed in Claim 1, **characterized in that** the powder catalyst used has an average particle diameter of 1 to 10 µm.

7. Process as claimed in Claim 1, **characterized in that** aliphatic alcohols with 1 to 5 carbon atoms are converted as alcohol components.
